# EUROPÄISCHE PATENTSCHRIFT

(11) **EP 1 379 546 B1**
(45) Veröffentlichungstag und Bekanntmachung des Hinweises auf die Patenterteilung: **13.06.2007**
(21) Anmeldenummer: 02737926.2
(22) Anmeldetag: 08.04.2002
(51) Int. Cl.: C07K 7/08, C07K 19/00, A61K 38/10, G01N 33/68, A61P 25/28

(54) **PEPTID ZUR DIAGNOSE UND THERAPIE DER ALZHEIMER-DEMENZ**
PEPTIDE FOR THE DIAGNOSIS AND THERAPY OF ALZHEIMER'S DISEASE
PEPTIDE SERVANT AU DIAGNOSTIC ET A LA THERAPIE DE LA MALADIE D'ALZHEIMER

(30) Priorität: 06.04.2001 DE 10117281
(43) Veröffentlichungstag der Anmeldung: 14.01.2004
(73) Patentinhaber: Leibniz-Institut für Altersforschung - Fritz-Lipmann-Institut e.V., 07745 Jena (DE)
(72) Erfinder: WILLBOLD, Dieter, 07751 Jena-Cospeda (DE); WIESEHAN, Katja, 07743 Jena (DE)
(74) Vertreter: Maiwald Patentanwalts GmbH
(86) Internationale Anmeldenummer: PCT/EP2002/003862
(87) Internationale Veröffentlichungsnummer: WO 2002/081505

(56) Entgegenhaltungen:
- WO-A-00/23465
- WO-A-00/52048
- WO-A-00/68263
- WO-A-01/07474
- TJERNBERG LO ET AL.: "Controlling Amyloid beta-Peptide Fibril Formation with Protease-stable Ligands" THE JOURNAL OF BIOLOGICAL CHEMISTRY, Bd. 272, Nr. 19, 9. Mai 1997 (1997-05-09), Seiten 12601-12605, XP002050230 in der Anmeldung erwähnt
- FINDEIS MA ET AL.: "Modified-Peptide Inhibitors of Amyloid beta-Peptide Polymerization" BIOCHEMISTRY, Bd. 38, 25. Mai 1999 (1999-05-25), Seiten 6791-6800, XP002143947
- ROTHBARD J.B.; GEFTER M.L.: 'Interactions between immunogenic peptides and MHC peptides' ANNUAL REVIEWS OF IMMUNOLOGY Bd. 9, 1991, Seiten 527 - 565
- WIESEHAN K. ET AL: 'Selection of D-Amino-Acid Peptides That Bind to Alzheimer's Disease Amyloid Peptide A.beta.1-42 by Mirror Image Phage Display' CHEMBIOCHEM Bd. 4, 2003, Seiten 748 - 753

### SEQUENZPROTOKOLL

<110> IMB Institut für molekulare Biotechnologie e.V.
<120> Peptid zur Diagnose und Therapie der Alzheimer-Demenz
<130> I7350
<140> PCT/EP02/03862
   <141> 2002-04-08
<150> DE 101 17 281.8
   <151> 2001-04-06
<160> 55
<170> PatentIn Ver. 2.1
<210> 1
   <211> 8
   <212> PRT
   <213> Homo sapiens
<220>
   <221> PEPTIDE
   <222> (1)..(8)
<400> 1
   Ser His Tyr Arg His Ile Ser Pro
    1               5
<210> 2
   <211> 12
   <212> PRT
   <213> Homo sapiens
<220>
   <221> PEPTIDE
   <222> (1)..(12)
<400> 2
   Gly Ile Ser Trp Gln Gln Ser His His Leu Val Ala
   1 5 10
<210> 3
   <211> 8
   <212> PRT
   <213> Homo sapiens
<220>
   <221> PEPTIDE
   <222> (1)..(8)
<400> 3
   Pro Arg Thr Arg Leu His Thr His
   1 5
<210> 4
   <211> 12
   <212> PRT
   <213> Homo sapiens
<220>
   <221> PEPTIDE
   <222> (1) .. (12)
<400> 4
   Gln Ser His Tyr Arg His Ile Ser Pro Ala Gln Val
   1 5 10
<210> 5
   <211> 12
   <212> PRT
   <213> Homo sapiens
<220>
   <221> PEPTIDE
   <222> (1)..(12)
<400> 5
   Gln Ser His Tyr Arg His Ile Ser Pro Asp Gln Val
   1 5 10
<210> 6
   <211> 11
   <212> PRT
   <213> Homo sapiens
<220>
   <221> PEPTIDE
   <222> (1) .. (11)
<400> 6
   Gln Ser His Tyr Arg His Ile Ser Pro Ala Arg
   1 5 10
<210> 7
   <211> 12
   <212> PRT
   <213> Homo sapiens
<220>
   <221> PEPTIDE
   <222> (1).. (12)
<400> 7
   Lys Ser His Tyr Arg His Ile Ser Pro Ala Lys Val
   1 5 10
<210> 8
   <211> 12
   <212> PRT
   <213> Homo sapiens
<220>
   <221> PEPTIDE
   <222> (1) .. (12)
<400> 8
   Arg Pro Arg Thr Arg Leu His Thr His Arg Asn Arg
   1 5 10
<210> 9
   <211> 12
   <212> PRT
   <213> Homo sapiens
<220>
   <221> PEPTIDE
   <222> (1)..(12)
<400> 9
   Arg Pro Arg Thr Arg Leu His Thr His Arg Thr Glu
   1 5 10
<210> 10
   <211> 12
   <212> PRT
   <213> Homo sapiens
<220>
   <221> PEPTIDE
   <222> (1).. (12)
<400> 10
   Lys Pro Arg Thr Arg Leu His Thr His Arg Asn Arg
   1 5 10
<210> 11
   <211> 42
   <212> PRT
   <213> Homo sapiens
<220>
   <221> PEPTIDE
   <222> (1)..(42)
   <223> beta-Amyloid-Peptid
<400> 11
<210> 12
   <211> 12
   <212> PRT
   <213> Homo sapiens
<220>
   <221> PEPTIDE
   <222> (1) .. (12)
<400> 12
   His His Gly His Ser Pro Asn Val Ser Gln Val Arg
   1 5 10
<210> 13
   <211> 12
   <212> PRT
   <213> Homo sapiens
<220>
   <221> PEPTIDE
   <222> (1)..(12)
<400> 13
   Gly Ser Phe Ser Thr Gln Val Gly Ser Leu His Arg
    1               5                  10
<210> 14
   <211> 11
   <212> PRT
   <213> Homo sapiens
<220>
   <221> PEPTIDE
   <222> (1) .. (11)
<400> 14
   His Thr Gly Thr Gln Ser Tyr Val Pro Arg Leu
    1               5                  10
<210> 15
   <211> 12
   <212> PRT
   <213> Homo sapiens
<220>
   <221> PEPTIDE
   <222> (1)..(12)
<400> 15
   Thr Leu Ala Tyr Ala Arg Ala Tyr Met Val Ala Pro
    1               5                  10
<210> 16
   <211> 12
   <212> PRT
   <213> Homo sapiens
<220>
   <221> PEPTIDE
   <222> (1) .. (12)
<400> 16
   Ala Thr Pro Gln Asn Asp Leu Lys Thr Phe Pro His
   1 5 10
<210> 17
   <211> 12
   <212> PRT
   <213> Homo sapiens
<220>
   <221> PEPTIDE
   <222> (1) **..** (12)
<400> 17
   Thr Gln Pro Glu Thr Asp Leu Leu Arg Val Gln Phe
   1 5 10
<210> 18
   <211> 11
   <212> PRT
   <213> Homo sapiens
<220>
   <221> PEPTIDE
   <222> (1) .. (11)
<400> 18
   Cys Ile Thr Trp Pro Pro Thr Gly Leu Thr Tyr
   1 5 10
<210> 19
   <211> 12
   <212> PRT
   <213> Homo sapiens
<220>
   <221> PEPTIDE
   <222> (1)..(12)
<400> 19
   Thr Phe Leu Glu Thr Gly Pro Ile Tyr Ala Asp Gly
   1 5 10
<210> 20
   <211> 12
   <212> PRT
   <213> Homo sapiens
<220>
   <221> PEPTIDE
   <222> (1)..(12)
<400> 20
   Leu Val Pro Pro Thr His Arg His Trp Pro Val Thr
   1               5                   10
<210> 21
   <211> 12
   <212> PRT
   <213> Homo sapiens
<220>
   <221> PEPTIDE
   <222> (1)..(12)
<400> 21
   Ala Pro Pro Gly Asn Trp Arg Asn Tyr Leu Met Pro
   1               5                   10
<210> 22
   <211> 12
   <212> PRT
   <213> Homo sapiens
<220>
   <221> PEPTIDE
   <222> (1)..(12)
<400> 22
   Asp Asn Tyr Ser Asn Tyr Val Pro Gly Thr Lys Pro
   1               5                   10
<210> 23
   <211> 12
   <212> PRT
   <213> Homo sapiens
<220>
   <221> PEPTIDE
   <222> (1) .. (12)
<400> 23
   Ser Val Ser Val Gly Met Lys Pro Ser Pro Arg Pro
   1 5 10
<210> 24
   <211> 12
   <212> PRT
   <213> Homo sapiens
<220>
   <221> PEPTIDE
   <222> (1)..(12)
<400> 24
   Ser Leu Pro Asn Pro Phe Ser Val Ser Ser Phe Gly
   1 5 10
<210> 25
   <211> 12
   <212> PRT
   <213> Homo sapiens
<220>
   <221> PEPTIDE
   <222> (1)..(12)
<400> 25
   Tyr Val His Asn Pro Tyr His Leu Pro Asn Pro Pro
   1 5 10
<210> 26
   <211> 12
   <212> PRT
   <213> Homo sapiens
<220>
   <221> PEPTIDE
   <222> (l)..(12)
<400> 26
   Cys Arg Arg Leu His Thr Tyr Ile Gly Pro Val Thr
   1 5 10
<210> 27
   <211> 12
   <212> PRT
   <213> Homo sapiens
<220>
   <221> PEPTIDE
   <222> (1)..(12)
<400> 27
   Gly Ala Thr Met Lys Lys Met Asp Asp His Thr Val
   1 5 10
<210> 28
   <211> 12
   <212> PRT
   <213> Homo sapiens
<220>
   <221> PEPTIDE
   <222> (1)..(12)
<400> 28
   Leu Gly Lys Thr Gln Lys Leu Ser Asp Ala His Ser
   1 5 10
<210> 29
   <211> 12
   <212> PRT
   <213> Homo sapiens
<220>
   <221> PEPTIDE
   <222> (1)..(12)
<400> 29
   Asp Asp Gln Ala Arg Pro Tyr Met Ala Tyr Gly Pro
   1 5 10
<210> 30
   <211> 12
   <212> PRT
   <213> Homo sapiens
<220>
   <221> PEPTIDE
   <222> (1)..(12)
<400> 30
   Gly Asp Thr Trp Val Asn Met Val Ser Met Val His
   1 5 10
<210> 31
   <211> 12
   <212> PRT
   <213> Homo sapiens
<220>
   <221> PEPTIDE
   <222> (1)..(12)
<400> 31
   Gly Tyr Thr Trp Val Asn Met Val Ser Met Val His
   1 5 10
<210> 32
   <211> 12
   <212> PRT
   <213> Homo sapiens
<220>
   <221> PEPTIDE
   <222> (1)..(12)
<400> 32
   Trp Thr Asn Thr Val Ala Arg Leu Ala Thr Pro Tyr
   1 5 10
<210> 33
   <211> 12
   <212> PRT
   <213> Homo sapiens
<220>
   <221> PEPTIDE
   <222> (1) .. (12)
<400> 33
   Gln Thr Gln Ala Leu Tyr His Ser Arg Gln Val His
   1 5 10
<210> 34
   <211> 12
   <212> PRT
   <213> Homo sapiens
<220>
   <221> PEPTIDE
   <222> (1)..(12)
<400> 34
   Asn Ser Gln Thr Gln Thr Leu His Leu Phe Pro His
   1 5 10
<210> 35
   <211> 12
   <212> PRT
   <213> Homo sapiens
<220>
   <221> PEPTIDE
   <222> (1)..(12)
<400> 35
   His Asn Thr Ser Ala Asn Ile Leu His Ser Ser His
   1 5 10
<210> 36
   <211> 12
   <212> PRT
   <213> Homo sapiens
<220>
   <221> PEPTIDE
   <222> (1)..(12)
<400> 36
   Ser His Ile Asn Pro Thr Ser Phe Trp Pro Ala Pro
   1 5 10
<210> 37
   <211> 12
   <212> PRT
   <213> Homo sapiens
<220>
   <221> PEPTIDE
   <222> (1)..(12)
<400> 37 <210> 38
   <211> 12
   <212> PRT
   <213> Homo sapiens
<220>
   <221> PEPTIDE
   <222> (1)..(12)
<400> 38 <210> 39
   <211> 12
   <212> PRT
   <213> Homo sapiens
<220>
   <221> PEPTIDE
   <222> (1)..(12)
<400> 39
<210> 40
   <211> 12
   <212> PRT
   <213> Homo sapiens
<220>
   <221> PEPTIDE
   <222> (1)..(12)
<400> 40
   Ser Thr Ser Val Tyr Pro Pro Pro Pro Ser Ala Trp
   1 5 10
<210> 41
   <211> 12
   <212> PRT
   <213> Homo sapiens
<220>
   <221> PEPTIDE
   <222> (1)..(12)
<400> 41
   Tyr Gly Leu Pro Thr Gln Ala Asn Ser Met Gln Leu
   1 5 10
<210> 42
   <211> 12
   <212> PRT
   <213> Homo sapiens
<220>
   <221> PEPTIDE
   <222> (1)..(12)
<400> 42
   His Asn Arg Thr Asp Asn Thr Tyr Ile Arg Pro Thr
   1 5 10
<210> 43
   <211> 12
   <212> PRT
   <213> Homo sapiens
<220>
   <221> PEPTIDE
   <222> (1)..(12)
<400> 43
   Leu Gln Gln Pro Leu Gly Asn Asn Arg Pro Asn Ser
   1 5 10
<210> 44
   <211> 12
   <212> PRT
   <213> Homo sapiens
<220>
   <221> PEPTIDE
   <222> (1) .. (12)
<400> 44
   Lys Pro Glu Asp Ser Ala Ala Tyr Pro Gln Asn Arg
   1 5 10
<210> 45
   <211> 12
   <212> PRT
   <213> Homo sapiens
<220>
   <221> PEPTIDE
   <222> (1)..(12)
<400> 45
   Arg Pro Glu Asp Ser Val Ile Lys Thr Gln Asn Thr
   1 5 10
<210> 46
   <211> 12
   <212> PRT
   <213> Homo sapiens
<220>
   <221> PEPTIDE
   <222> (1)..(12)
<400> 46
   Arg Ala Ala Asp Ser Gly Cys Ile Pro Thr Lys His
   1 5 10
<210> 47
   <211> 12
   <212> PRT
   <213> Homo sapiens
<220>
   <221> PEPTIDE
   <222> (1) .. (12)
<400> 47
   Arg Pro Arg Thr Arg Leu His Thr His Arg Asn Thr
   1 5 10
<210> 48
   <211> 12
   <212> PRT
   <213> Homo sapiens
<220>
   <221> PEPTIDE
   <222> (1)..(12)
<400> 48
   Arg Pro Arg Thr Arg Leu His Thr His Arg Asn Val
   1 5 10
<210> 49
   <211> 12
   <212> PRT
   <213> Homo sapiens
<220>
   <221> PEPTIDE
   <222> (1)..(12)
<400> 49
   Lys Pro Arg Thr Arg Ile His Thr His Arg Asn Arg
   1               5                   10
<210> 50
   <211> 12
   <212> PRT
   <213> Homo sapiens
<220>
   <221> PEPTIDE
   <222> (1)..(12)
<400> 50
   Arg Pro Arg Thr Arg Leu His Thr His Thr Asn Arg
   1               5                   10
<210> 51
   <211> 12
   <212> PRT
   <213> Homo sapiens
<220>
   <221> PEPTIDE
   <222> (1)..(12)
<400> 51
   Arg Pro Arg Thr Arg Leu His Thr His Arg Lys Gln
   1               5                   10
<210> 52
   <211> 12
   <212> PRT
   <213> Homo sapiens
<220>
   <221> PEPTIDE
   <222> (1)..(12)
<400> 52
   Arg Pro Arg Thr Arg Leu His Thr Leu Arg Asn Arg
   1 5 10
<210> 53
   <211> 12
   <212> PRT
   <213> Homo sapiens
<220>
   <221> PEPTIDE
   <222> (1)..(12)
<400> 53
   Arg Arg Arg Ser Pro Leu His Thr His Arg Asn Arg
   1 5 10
<210> 54
   <211> 12
   <212> PRT
   <213> Homo sapiens
<220>
   <221> PEPTIDE
   <222> (1)..(12)
<400> 54
   Leu Arg Ser Pro Arg Gln Arg Arg Ile Pro Arg Ile
   1 5 10
<210> 55
   <211> 12
   <212> PRT
   <213> Homo sapiens
<220>
   <221> PEPTIDE
   <222> (1)..(12)
<400> 55
   Arg Lys Arg Gln Leu Arg Met Thr Thr Pro Arg Pro
   1 5 10

## Beschreibung

Die Erfindung betrifft ein Peptid, das mit hoher Bindungsaffinität an das β-Amyloid-Peptid bindet, ein Verfahren zur Herstellung solcher Peptide sowie die Verwendung dieser Peptide zur Diagnose und Therapie der Alzheimer-Demenz.

Die Alzheimersche Krankheit ist die häufigste Form der Altersdemenz. Sie beginnt mit geringen Gedächtnisstörungen und führt im weiteren Krankheitsverlauf zum Verlust großer Teile des Gedächtnisses, zur Verminderung des Sprachschatzes auf wenige Wörter, zu räumlicher und zeitlicher Desorientierung, zur Unfähigkeit zu stehen und gehen, sowie häufig zu Harn- und Stuhlinkontinenz. Die statistische Auswertung der Epidemiologie der Alzheimer-Demenz zeigt, dass das Alter der größte Risikofaktor ist, und dass jeder, der nur alt genug wird, diese Krankheit bekommen wird. Meist wird die Krankheit erst nach dem 65. Lebensjahr manifest, zuweilen jedoch auch schon früher, beispielsweise um das 50 Lebensjahr. Die Alzheimer-Krankheit ist deshalb insbesondere für westliche Industriestaaten mit einer hohen Lebenserwartung ein immer größer werdendes soziales und finanzielles Problem.

Als zuverlässigste Diagnose der Alzheimer-Demenz gilt die post mortem-Identifizierung von sogenannten "Amyloid-Plaques" im Gehirn der jeweiligen Person. Diese Amyloid-Plaques sind im wesentlichen der einzig bisher bekannte Zusammenhang zwischen pathologischen Befunden und Krankheitssymptomen. Der Hauptbestandteil dieser Plaques sind Aggregate des β-Amyloid-Peptids bzw. Aβ-Peptids bzw. βA4-Peptids, das 39 bis 43 und üblicherweise 40 oder 42 Aminosäurereste umfasst. Ob die Plaques eine Folge der Krankheit sind oder umgekehrt die Krankheitssymptome auf dem Vorhandensein der Plaques beruhen, ist noch umstritten. Das letztere Modell, die sog. "Amyloid-Hypothese", wurde in den letzten Jahren immer vollständiger und konsistenter, obwohl ein endgültiger Beweis oder Gegenbeweis bis jetzt nicht erbracht werden konnte (D.J. Selkoe, Science 1997, 275, 630 - 631).

Das Aβ-Peptid entsteht aus dem Aβ-Vorläuferprotein APP. APP ist ein Transmembranprotein, das durch verschiedene Proteasen, die als α-, β- oder γ-Sekretasen bezeichnet werden, gespalten wird. Durch die Aktivität von β- und γ-Sekretasen entsteht das Aβ-Peptid. Die bisherige Forschung ist darauf gerichtet, einen Inhibitor gegen diese Proteasen zu finden. Derartige Inhibitoren weisen jedoch erhebliche Nachteile auf, da die APP-spaltende Aktivität dieser Proteasen nicht deren einzige Funktion ist und somit die Hemmung der Enzyme zu drastischen Nebenwirkungen führen kann.

Es besteht somit ein Bedarf an Verbindungen, die zur Diagnose und/oder zur Therapie von Alzheimer eingesetzt werden können und keine bzw. möglichst geringe Nebenwirkungen für den Patienten aufweisen.

Wünschenswert wäre es insbesondere, Verbindungen zu finden, die an das Aβ-Peptid binden und somit die Aggregation von Aβ hemmen. Zum Auffinden derartiger Verbindungen bieten sich Technologien an, die es ermöglichen, eine sehr große Zahl, beispielsweise 10⁶ bis 10¹², von chemisch unterschiedlichen Molekülen, eine sogenannte Bibliothek, sehr schnell und einfach nach bestimmten Eigenschaften zu durchsuchen bzw. zu "screenen". Dabei werden chemisch-synthetische Bibliotheken und "biologische", d.h. sich selbst replizierende Bibliotheken, unterschieden. Chemisch-synthetische Bibliotheken bieten ein nahezu unbegrenztes Repertoire an Einzelbausteinen, ermöglichen jedoch nur das Screening einer begrenzten Zahl an verschiedenen Molekülen. Dagegen wird durch biologische Bibliotheken ein sehr schnelles Screening von bis zu 10¹² verschiedenen Molekülen in einem Arbeitsschritt gewährleistet. Derartige biologische Bibliotheken sind jedoch auf die Kombination von "natürlichen" Bausteinen beschränkt, wie beispielsweise Ribonuklein- und Desoxyribonukleinsäuren und L-Aminosäuren.

Bei der direkten Anwendung in vivo von Substanzen, die durch biologisches Screening erhalten wurden und somit beispielsweise aus L-Aminosäuren bestehen, ergeben sich ferner Nachteile dadurch, dass sie äußerst empfindlich gegenüber Proteasen sind und zudem fast immer auch eine heftige Immunantwort auslösen, wenn sie an tierischen Modellen oder für die Behandlung von Menschen verwendet werden. Das herkömmliche Peptid-Phagendisplay (Smith, Science 1985, 228, 1315-1317) ist somit in dieser Hinsicht keine ausreichende Methode zur Identifizierung von Verbindungen, die an das Aβ-Peptid binden.

Im Stand der Technik sind bereits Verbindungen beschrieben, die die in vitro-Aggregation von Aβ inhibieren bzw. dessen toxische Effekte vermindern. So ist bekannt, dass Kongorot an Aβ-Aggregate bindet, und deren toxische Wirkung inhibiert (Lorenzo und Yankner, Proc. Natl. Acad. Sci. USA 1994, 91,12243 - 12247). Ferner wurden von Tjernberg et al. (J. Biol. Chem. 1996, 271, 8545 - 8548) Peptide offenbart, die kleinen Abschnitten des Aβ-Peptids entsprechen und dessen Aggregation in vitro hemmen können. Salomon et al., Biochemistry 1996, 35, 13568 - 13578, haben die Bindung von Nikotin an helikales Aβ und die Inhibierung der Aβ-Aggregation gezeigt. Für Hexadecyl-N-methylpiperidiniumbromid und Rifampicin wurde ebenfalls eine die Aβ-Fibrillenbildung inhibierende und anticytotoxische Wirkung mit einer Dissoziationskonstante im µM- bis mM-Bereich berichtet (Tomiyama et al., J. Biol. Chem. 1996, 271, 6839 - 6844). Solomon et al., Proc. Natl. Acad. Sci. USA 1997, 94, 4109 - 4112 haben ferner Antikörper beschrieben, die gegen den N-terminalen Teil (Positionen 1 bis 28) von Aβ gerichtet sind. Diese Antikörper konnten die Toxizität von Aβ reduzieren und die Aggregation von Aβ verhindern und sogar rückgängig machen.

Ghanta et al., J. Biol. Chem. 1996, 271, 29525 - 29528, beschreiben die Synthese eines Peptids, welches aus zwei Teilen besteht. Der N-terminale Teil entspricht den Aminosäuren 15 bis 25 von Aβ, der C-terminale Teil besteht aus sechs aufeinander folgenden Lysinen. Der Aβ₁₅₋₂₅-Teil soll an Aβ binden, während der Hexalysin-Teil die Fibrillenbildung durch dessen Wasserlöslichkeit stören soll. Dieses Konstrukt ist in der Lage, die Aggregation von Aβ zu verlangsamen und dessen Cytotoxizität zu reduzieren.

Alle vorstehend beschriebenen Substanzen, welche die Aggregation von Aβ inhibieren, weisen jedoch den erheblichen Nachteil auf, dass sie in vivo leicht abgebaut werden, möglicherweise eine Immunreaktion auslösen und/oder zu unspezifisch wirken und dadurch zumindest langfristig schwere Nebenwirkungen auslösen.

Tjernberg et al., J. Biol. Chem. 1997, 272, 12601 - 12605, haben ferner ein Proteaseresistentes Pentapeptid beschrieben, das durch Screening einer chemisch synthetisierten Bibliothek aus D-Aminosäuren erhalten wurde. Dieses Peptid scheint an Aβ zu binden und die Bildung von Plaques zu inhibieren. Derartig kleine Peptide, wie sie durch Screening von herkömmlicherweise eher kleinen chemisch synthetischen Bibliotheken gefunden werden, sind jedoch nicht in der Lage, die erforderliche Affinität und insbesondere die gewünschte Spezifität für Aβ zu zeigen.

Es besteht somit insbesondere ein Bedarf an Aß-Inhibitoren, die eine Affinität für Aβ nahe dem physiologisch wichtigen nanomolaren und subnanomolaren Bereich aufweisen. Neben der hohen Affinität ist es ferner von großer Bedeutung, dass ein Wirkstoff für die Alzheimer-Therapie eine hohe Spezifität für das Aβ-Peptid aufweist, da davon auszugehen ist, dass eine pharmazeutische Zusammensetzung, die die Bildung von Aβ-Plaques hemmen und ggf. revertieren soll, über einen Zeitraum von unter Umständen mehreren Jahrzehnten verabreicht werden muß und deshalb frei von jeder Art von Nebenwirkungen sein sollte.

Aufgabe der vorliegenden Erfindung ist somit die Bereitstellung von Verbindungen, die zur zuverlässigen Diagnose der Alzheimer-Krankheit auch bei lebenden Patienten geeignet sind und/oder die zur Therapie der Alzheimer-Demenz eingesetzt werden können, ohne dass sie Nebenwirkungen aufweisen, die das Wohlbefinden des Patienten wesentlich einschränken. Insbesondere ist es eine wesentliche Aufgabe der vorliegenden Erfindung, für die Diagnose und/oder Therapie der Alzheimer-Demenz geeignete Verbindungen bereitzustellen, die bei einem potentiellen Einsatz in Tiermodellen bzw. im Menschen keine oder zumindest eine verminderte Protease-Sensitivität und ferner eine deutlich reduzierte Immunogenität aufweisen.

Weitere Aufgaben ergeben sich aus der folgenden Beschreibung.

Oben genannte Aufgaben werden durch die Merkmale der unabhängigen Schutzansprüche gelöst.

Vorteilhafte Ausgestaltungen sind in den Unteransprüchen angegeben.

Es wurden jetzt überraschenderweise Peptide gefunden, die mit hoher Bindungsaffinität und hoher Spezifität an Monomere und/oder Oligomere und/oder Fibrillen des β-Amyloid-Peptids (im folgenden auch als Aβ-Peptid oder Aβ bezeichnet) binden und im wesentlichen aus D-Aminosäuren bestehen. Insbesondere bindet ein erfindungsgemäßes Peptid an Monomere und/oder Oligomere und/oder Fibrillen des β-Amyloid-Peptid mit einer Dissoziationskonstante (im folgenden auch als K_{D}-Wert bezeichnet) von höchstens 10 µM, vorzugsweise von höchstens 8 µM, mehr bevorzugt von höchstens 6 µM, besonders bevorzugt von höchstens 4 µM und am meisten bevorzugt von höchstens 1 µM. Ganz besonders bevorzugt sind erfindungsgemäße Peptide, die mit einem K_{D}-Wert im nanomolaren bzw. subnanomolaren Bereich an das β-Amyloid-Peptid binden.

Die Dissoziationskonstante bzw. der K_{D}-Wert berechnet sich aus dem Quotienten von Dissoziationsrate und Assoziationsrate des β-Amyloid-Peptids und gibt das Gleichgewicht zwischen freier und gebundener Form wieder. Die

Dissoziationskonstante kann somit durch alle Messmethoden bestimmt werden, mit denen das Verhältnis von freier zu gebundener Form eines Substrats bzw. Monomers zugänglich ist, beispielsweise durch Fluoreszenztitration, Oberflächenplasmonenresonanz, ELISA, Radioimmunoassays und spektroskopische Verfahren wie NMR. Weitere Verfahren zur Bestimmung der Dissoziationskonstante sind dem Fachmann bekannt.

Das erfindungsgemäße, im wesentlichen aus D-Aminosäuren bestehende Peptid ist in einer Ausführungsform der vorliegenden Erfindung aufgrund seiner hohen Affinität und Spezifität für Oligomere bzw. Fibrillen von Aβ in der Lage, die bei der Alzheimer-Demenz auftretenden Aβ-Plaques bzw. Aggregate aufzulösen oder zumindest deren weiteres Wachstum zu verhindern. Ferner ermöglicht das erfindungsgemäße Peptid, die Cytotoxizität von Aβ zu inhibieren. Neben diesen therapeutischen Eigenschaften ist das erfindungsgemäße Peptid hervorragend für diagnostische Tests zum Nachweis von Aβ geeignet.

Bei einer alternativen Ausführungsform ist das erfindungsgemäße, im wesentlichen aus D-Aminosäuren bestehende Peptid aufgrund seiner hohen Affinität und Spezifität für Monomere von Aβ in der Lage, die Oligomerisierung von Aβ und damit die Entstehung von Aβ**-**Plaques zu inhibieren. Damit ist das erfindungsgemäße Peptid zum Einsatz in der Vorbeugung von Alzheimer prädestiniert.

Zudem weist das erfindungsgemäße D-Peptid gegenüber den herkömmlichen Peptiden aus L-Aminosäuren die herausragende Eigenschaft auf, dass es in in vivo-Anwendungen wesentlich weniger immunogen ist und gleichzeitig deutlich resistenter gegenüber Proteasen ist.

Im wesentlichen aus D-Aminosäuren bestehend bedeutet im Rahmen der vorliegenden Erfindung, dass die an der Bindung an das Aβ-Peptid beteiligten

Aminosäuren des erfindungsgemäßen Peptids im wesentlichen D-Aminosäuren sind, d.h. mindestens 75%, vorzugsweise mindestens 80%, besonders bevorzugt mindestens 85% und am meisten bevorzugt 100% der an der Bindung an das Aβ-Peptid beteiligten Aminosäuren D-Aminosäuren sind.

Daneben kann es vorteilhaft sein, dass an die im wesentlichen, vorzugsweise ausschließlich aus D-Aminosäuren bestehende Bindungsregion des erfindungsgemäßen Peptids weitere Gruppen, beispielsweise L-Aminosäuren, Aminosäureanaloga und dergleichen angefügt werden. Insbesondere kann eine Verknüpfung der im wesentlichen aus D-Aminosäuren bestehenden Bindungsregion des erfindungsgemäßen Peptids mit gut, vorzugsweise sehr gut wasserlöslichen Substanzen wie beispielsweise Zuckerresten die Amyloid-Plaque-lösende Wirkung wesentlich erhöhen. Für diese Zwecke geeignete'wasserlösliche Gruppen, insbesondere Zuckerreste, Glucuronsäuren, Sulfatreste, Serin, Glycin, Aspartat und dergleichen, sowie die Art und Weise der Verknüpfung dieser Gruppen mit dem erfindungsgemäßen Peptid sind dem Fachmann bekannt.

Ferner können die erfindungsgemäßen Peptide mit herkömmlichen Antikörpern oder Fragmenten davon auf übliche Weise verknüpft werden, um bei einer therapeutischen Anwendung des erfindungsgemäßen Peptids Bestandteile des Immunsystems zu aktivieren und dadurch Aβ oder ganze Aβ-Plaques dem Abbau beispielsweise durch Makrophagen, Mikrogliazellen usw. zuzuführen.

Weitere Gruppen, mit denen die im wesentlichen aus D-Aminosäuren bestehende Bindungsregion des erfindungsgemäßen Peptids verknüpft werden kann, ergeben sich aus der Verwendung der erfindungsgemäßen Peptide zur Diagnose der Alzheimer-Demenz mittels Bild- oder anderen Signal-gebenden Verfahren wie PET (Positronen-Emissions-Tomographie), Kernspin-Tomographie, Computertomographie, SPECT (Single-Photon-Computer-Tomographie) und Nah-Infrarot-Detektion. Üblicherweise für derartige Imaging-Techniken verwendetet Gruppen sind dem Fachmann ebenso wie die Art und Weise der Verknüpfung dieser Gruppen mit dem erfindungsgemäßen Peptid ohne weiteres geläufig. Beispiele hierfür sind Gadoliniumkomplexe, Iod, Technetium; Thallium, Fluor und dergleichen.

Schließlich können an die erfindungsgemäßen Peptide funktionelle Gruppen synthetisch angefügt werden, die das Wiederausscheiden der erfindungsgemäßen Peptide, frei oder gebunden an Aβ, aus dem Körper beschleunigen können. Beispiele hierfür sind unter anderem Glucuronsäure und Sulfatgruppen.

Ferner ist es bevorzugt, dass das erfindungsgemäße Peptid 4 bis 30, vorzugsweise 4 bis 20, mehr bevorzugt 5 bis 18, noch mehr bevorzugt 6 bis 15 und besonders bevorzugt 7 bis 9 Aminosäuren umfasst. Peptide in diesem Sequenzlängenbereich sind klein genug, um beispielsweise als Kontrastmittel bei der Detektion von Amyloidplaques in lebenden Individuen eingesetzt werden zu können.

Bei einer Ausführungsform der vorliegenden Erfindung umfasst das Peptid das im wesentlichen aus D-Aminosäuren bestehende Aminosäure-Sequenzmotiv SHYRHISP. Bei einer weiteren Ausführungsform der vorliegenden Erfindung umfasst das Peptid das im wesentlichen aus D-Aminosäuren bestehende Sequenzmotiv GISWQQSHHLVA Bei einer weiteren Ausführungsform der vorliegenden Erfindung umfasst das Peptid das im wesentlichen aus D-Aminosäuren bestehende Sequenzmotiv PRTRLHTH.

Peptide, die die vorstehend genannten Sequenzmotive umfassen, weisen eine sehr hohe Bindungsaffinität an das β-Amyloid-Peptid auf.

Besonders bevorzugt wird das Peptid ausgewählt aus der Gruppe, bestehend aus Peptiden mit den D-Aminosäure-Sequenzen:
a) QSHYRHISPAQV;
b) QSHYRHISPDQV;
c) QSHYRHISPAR;
d) KSHYRHISPAKV;
e) RPRTRLHTHRNR;
f) RPRTRLHTHRTE; und
g) KPRTRLHTHRNR;

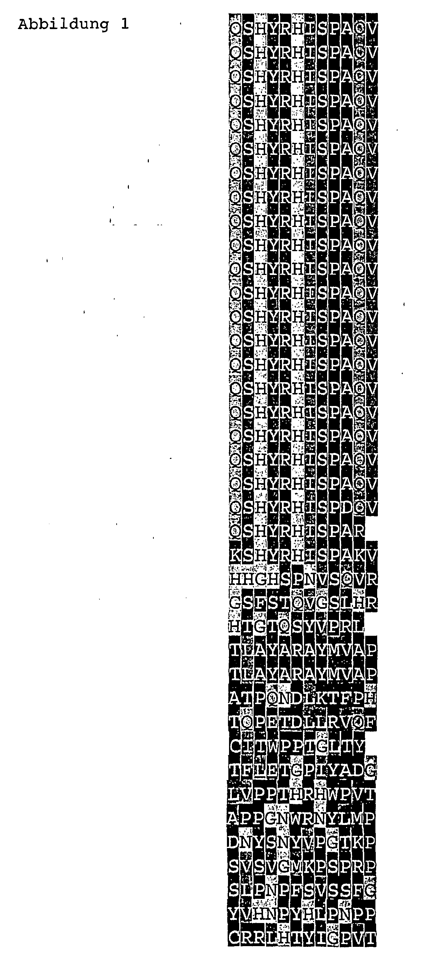

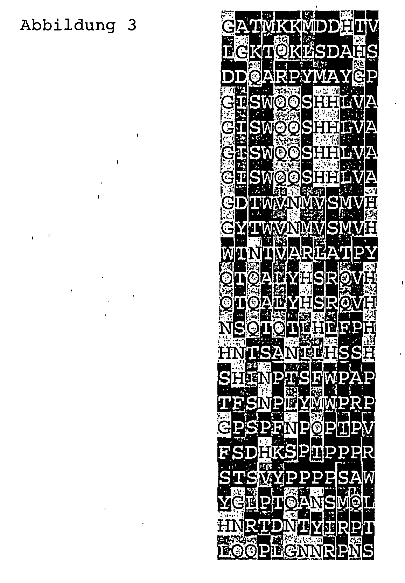

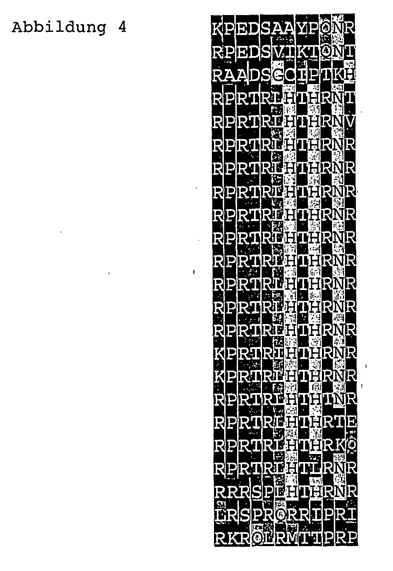

Weitere Beispiele für bevorzugte erfindungsgemäße Peptide sind in den Abbildungen 1, 3 und 4 gezeigt sofern sie unter den Schutz des unabhängigen Anspruchs 1 fallen. Dabei sind die in den Abbildungen 1 und 3 aufgeführten Peptide insbesondere zur Diagnose der Alzheimer-Demenz geeignet, während die in Abbildung 4 gezeigten Peptide insbesondere zur Vorbeugung und/oder Therapie der Alzheimer-Demenz eingesetzt werden können. Die in den Abbildungen 1, 3 und 4 gezeigten erfindungsgemäßen Peptide bestehen im wesentlichen, vorzugsweise ausschließlich aus D-Aminosäuren.

Weiterhin wird ein Verfahren zur Identifizierung bzw. Herstellung von Peptiden, die an das Aβ-Peptid binden, beschrieben.

Eine derartige Selektion ist auf direktem Weg nur mit chemischen Bibliotheken möglich. Um die im Vergleich zu chemischen Bibliotheken weit umfangreicheren biologischen Bibliotheken für das Screening verwenden zu können, wurde bei der vorliegenden Erfindung jedoch die Tatsache ausgenützt, dass zwei Moleküle in gleicher Weise wechselwirken und aneinander binden wie ihre jeweiligen Stereoisomere. Dabei verhält sich ein Komplex zum anderen wie Bild und Spiegelbild. Selektiert man nun eine biologische Bibliothek gegen ein Spiegelbild des Targets, d.h. im vorliegenden Fall gegen ein β-Amyloid-Peptid bestehend aus D-Aminosäuren, so wird nach dem Selektionsverfahren ein Peptid erhalten, das aus L-Aminosäuren besteht (im folgenden auch als L-Peptid bezeichnet). Das Spiegelbild dieses L-Peptids, d.h. ein Peptid, das aus D-Aminosäuren mit gleicher Sequenz besteht (im folgenden auch als D-Peptid bezeichnet), bindet nun auf die selbe Weise an das Target wie das L-Peptid an das "Mirror Target".

Erfindungsgemäß wird somit ein Verfahren zur Herstellung bzw. Selektion von Peptiden bereitgestellt, die mit hoher Bindungsaffinität und -spezifität an das β-Amyloid-Peptid binden, umfassend die folgenden Schritte:
a) Synthese eines β-Amyloid-Peptids, bestehend aus D-Aminosäuren;
b) Screening von biologischen Peptid-Bibliotheken mittels Phagendisplay gegen die β-Amyloid-Sequenz aus Schritt a);
c) Identifizierung der an die β-Amyloid-Sequenz bindenden Peptide; und
d) Synthese der in Schritt c) selektierten Peptide mit der entsprechenden Aminosäuresequenz aus D-Aminosäuren.

Das in Schritt a) hergestellt β-Amyloid Peptid umfasst die Aminosäuren 1 bis 42 der bekannten β-Amyloid-Sequenz oder Fragmente davon.

Vorzugsweise werden bei dem Verfahren solche Peptide hergestellt bzw. selektiert, die mit einem K_{D}-Wert von höchstens 10 µM, vorzugsweise von höchstens 8 µM, mehr bevorzugt von höchstens 6 µM, besonders bevorzugt von höchstens 4 µM und am meisten bevorzugt von höchstens 1 µM an Monomere und/oder Oligomere und/oder Fibrillen des β-Amyloid-Peptids binden.

Bei einer bevorzugten Ausführungsform des Verfahrens erfolgt das Screening in Schritt b) mittels Phagen-Display. Dieses als Spiegelbild-Phagendisplay (Schumacher et al., Science 1996, 271, 1854 - 1857) bezeichnete Verfahren bietet somit die Möglichkeit, mit hoher Bindungsaffinität und -spezifität an das Aβ**-**Peptid bindende Peptide zu identifizieren, die aus D-Aminosäuren bestehen, und eine stark verminderte Proteaseempfindlichkeit sowie ein reduziertes immunogenes Potential aufweisen.

Aufgrund der extremen Aggregationsempfindlichkeit von Aβ war ein erfolgreiches Screening von biologischen Peptid-Bibliotheken gegen die β-Amyloid-Sequenz nicht zu erwarten. Insbesondere sind die experimentellen Parameter bei Schritt b) des vorstehend beschriebenen erfindungsgemäßen Verfahrens in Abhängigkeit davon einzustellen, ob nach dem biologischen Screening ein Peptid erhalten werden soll, das bevorzugt an Monomere oder Oligomere bzw. Fibrillen bindet.

Falls das erfindungsgemäße Peptid für die Diagnose und/oder Therapie von Alzheimer eingesetzt werden soll, ist es insbesondere erwünscht, dass das durch das biologische Screening selektierte Peptid vorzugsweise an Oligomere bzw. Fibrillen von Aβ bindet. Um dies zu gewährleisten, wird das β-Amyloid-Peptid bei dem erfindungsgemäßen Verfahren zunächst in Lösungsbedingungen überführt, die dessen kontrollierte Aggregation ermöglichen. Eine unkontrollierte Aggregation von Aβ wird durch Verwendung eines geeigneten Lösungsmittels, insbesondere von DMSO (Dimethylsulfoxid) verhindert. Anschließend wird in Schritt b) des erfindungsgemäßen Verfahrens die Peptidbibliothek zugegeben und schließlich die gebildeten Peptid/D-Aβ-Komplexe beispielsweise an Streptavidin immobilisiert.

Falls das erfindungsgemäße Peptid zur Vorbeugung der Alzheimer-Demenz eingesetzt werden soll, ist es insbesondere erwünscht, durch das biologische Screening Peptide zu selektieren, die bevorzugt an Aβ-Monomere binden. In diesem Fall wird bei einer bevorzugten Ausführungsform des erfindungsgemäßen Verfahrens D-Aβ zunächst in vorzugsweise geringer Konzentration beispielsweise an Streptavidin immobilisiert. Dies verhindert die Aggregation von Aβ vollständig, da alle Aβ-Moleküle einzeln immobilisiert sind. Anschließend erfolgt die Zugabe der Peptidbibliothek und Selektion der an D-Aβ-Monomere bindenden Peptide.

Ob das durch das beschriebene Verfahren selektierte Peptid an Monomere oder Oligomere bzw. Fibrillen bindet, ist somit durch geeignete Wahl der Schritte und Bedingungen im Verlauf des Selektionsverfahrens einstellbar.

Die in Schritt a) des vorstehend beschriebenen Verfahrens synthetisierten D-Peptide wurden mit spektroskopischen Methoden wie NMR-Spektroskopie und Cirkulardichroismus-Spektroskopie untersucht. So wurden beispielsweise alle Protonen von L-Aβ bzw. D-Aβ mittels NMR-Spektroskopie identifiziert. Aufgrund der im wesentlichen identischen NMR-Spektren von L-Aβ und D-Aβ sowie der Tatsache, dass die entsprechenden CD-Spektren bei umgekehrten Vorzeichen identisch sind, wurde gewährleistet, dass es sich bei dem in Schritt a) hergestellten D-Aβ um das Spiegelbild von L-Aβ handelt.

Das biologische Screening in Schritt b) des vorstehend beschriebenen, Verfahrens kann alternativ auch mit Plasmid-Display (Schatz et al., Meth. Enzymol. 1996, 267, 171), Ribosomen-Display (Hanes und Plückthun, PNAS, 1997, 94, 4937-4942), Polysomen-Display (Mattheakis et al., PNAS, 1994, 91, 9022-9026), Baculoviren-Display (Ernst et al., Nucl. Acids Res. 1998, 26, 1718-1723), Bakterien-Display (Stahl und Uhlen, Trends Biotech. 1997, 15, 185-192) und/oder in vivo-Display erfolgen.

Ein weiterer Gegenstand der vorliegenden Erfindung ist die Verwendung des erfindungsgemäßen Peptids zur Diagnose der Alzheimer-Demenz bei lebenden Individuen, insbesondere durch diagnostisches Imaging der Amyloid-Plaques in lebenden Tieren oder Menschen. Somit ist durch die Verwendung der erfindungsgemäßen Peptide für diagnostisches Imaging bei Tieren auch eine wesentliche Verminderung der Anzahl an Tierversuchen möglich. Tierversuche mit potentiellen Wirkstoffen gegen die Alzheimer-Demenz beinhalten nämlich üblicherweise als essentiellen Schritt die Kontrolle des Einflusses der zu testenden Substanz auf die Entwicklung von Plaques im Laufe der Zeit. Dazu ist es bislang erforderlich gewesen, Tierversuche mit einer großen Zahl an Versuchstieren durchzuführen, da zu bestimmten Zeitpunkten immer wieder Tiere getötet werden müssen, um Zahl und Größe der Plaques zu kontrollieren. Durch Verwendung der erfindungsgemäßen Peptide ist eine Kontrolle am lebenden Tier möglich, so dass die Tierversuche mit potentiellen Wirkstoffen gegen Alzheimer mit einer deutlich verringerten Anzahl an Versuchstieren durchgeführt werden können.

Bei einer bevorzugten Ausführungsform der vorliegenden Erfindung werden die Peptide gebunden an eine geeignete Substanz als Kontrastmittel zur Detektion von Amyloid-Plaques mittels Computertomographie (CT) eingesetzt. Alternativ werden die erfindungsgemäßen Peptide vorzugsweise mit einer radioaktiven Markierung versehen. So können beispielsweise mit Hilfe der sogenannten Positronen-Emissions-Tomographie (PET) mit Positronen-emittierenden Radionukliden markierte erfindungsgemäße Peptide injiziert werden und die emittierte γ-Strahlung in Tomogrammen erfaßt werden. Nützliche Positronenemitter sind ¹¹C, ²²Na, ⁵⁸Co und dergleichen.

Weitere Alternativen zur Diagnose der Alzheimer-Demenz bei lebenden Individuen mittels der erfindungsgemäßen Peptide sind Kernspintomographie (MRI, magnetic resonance imaging), SPECT (single photon emission computer tomographie) und/oder Nah-Infrarot-Detektion (NIR).

Aufgrund der Fähigkeit der erfindungsgemäßen Peptide zur spezifischen Wechselwirkung mit dem Plaque-Aufbau von Aβ beziehungsweise zur Inhibierung der Toxizität von Aβ können die erfindungsgemäßen Peptide ferner zur Vorbeugung und/oder zur Therapie der Alzheimer-Demenz verwendet werden.

Da die erfindungsgemäßen D-Peptide verglichen mit aus L-Aminosäuren bestehenden Peptiden eine stark reduzierte Protease-Empfindlichkeit und ein vermindertes immunogenes Potential aufweisen, sind sie ebenfalls geeignet, um den Zusammenhang zwischen pathologischen Befunden und Krankheitssymptomen bei der Alzheimer-Demenz zu untersuchen, insbesondere um die Hypothese des "Amyloid-Modells" zu verifizieren.

Die vorliegende Erfindung wird in den nachfolgenden Beispielen veranschaulicht, ohne sie in irgendeiner Weise einzuschränken.

### Beispiele

Folgende Peptide wurden als reversed phase-HPLC-gereinigte Produkte von Jerini Biotools, Berlin, Deutschland bezogen:
- Biotinyl-DAEFRHDSGYEVHHQKLVFFAEDVGSNKGAIIGLMVGGVVIA (alle Aminosäuren sind L-Enantiomere, im folgenden auch als L-Aβ bezeichnet);
- D-Aβ (dieselbe Sequenz wie L-Aβ, aber alle Aminosäuren sind D-Enantiomere);
- QSHYRHISPAQV (alle Aminosäuren sind D-Enantiomere) mit einem über ein L-Lysin am C-Terminus gebundenen Fluoresceinyl-Rest (im folgenden auch als D-Pep bezeichnet).

Die Identität von L-Aβ und D-Aβ wurde durch MALDI-TOF-MS (Matrix-assistierte Laser-desorptions-Ionisierungs-Flugzeitmassen-Spektrometrie) sowie durch NMR und Cirkulardichroismus-Spektroskopie bestätigt.

Die DNA-Sequenzen wurden unter Verwendung des Big Dye Terminator Cycle Sequencing Kit von PE Applied Biosystems (Warrington, Groß Britannien) mit Ampli Taq DNA-Polymerase bestimmt.

Die Fluoreszenz-Messungen wurden bei 298 K auf einem SLM-Aminco-Fourier Transform-Spektrofluorometer 48000 MHF (SLM Instruments Inc., USA) oder einem LS502-Fluorometer (Perkin Elmer) in Standardfluoreszenz-Zellen aus SUPRASIL-Quartzglas (10 x 10 mm, Hellma, Deutschland) durchgeführt. Die Fluoreszenz wurde bei ständigem Rühren in Buffer (PBS mit 1 mM Natriumdodecylsulfat) unter Verwendung von Anregungs- und Emissionswellenlängen von 495 bzw. 520 nm gemessen. Dazu wurden geeignete Mengen von L-Aβ in Hexafluoroisopropanol (HFP) gelöst. Als Kontrolle wurden dieselben Titrationen mit HFP in Abwesenheit von L-Aβ durchgeführt.

### In vitro-Selektionen von an D-Aβ bindenden Peptiden:

### Selektionsverfahren A:

Zur Durchführung eines Phagendisplays wurde eine Vorratslösung aus 250 µM D-Aβ in DMSO hergestellt. 2x10¹¹ Phagen (Phagendisplay-12 Phagedisplay Peptide Library Kit, New England Biolabs, Beverly, Madison, USA) in 0,8 ml TBST (50 mM Tris-HCl pH 7,5, 150 mM NaCl, 0,1 Vol.-%, Tween-20 (Sigma, Deisenhofen)) enthaltend 0,1 Gew.-% BSA (Sigma) wurden 1:100 mit 250 µM D-Aβ in DMSO gemischt, was zu einer Endkonzentration von 2,5 µM D-Aβ führte.

Nach 10 Min. wurde die Phagen-Peptid-Suspension in ein mit Streptavidin beschichtetes Röhrchen (Boehringer Mannheim) überführt und für 15 Min. bei Raumtemperatur leicht geschüttelt.

### Selektionsverfahren B:

Im Unterschied zu dem Selektionsverfahren A, bei der zunächst das β-Amyloid-Peptid mit den Phagen gemischt wurde und anschließend die Mischung immobilisiert wurde, wurde bei dem Selektionsverfahren B zunächst das β-Amyloid-Peptid immobilisiert und erst danach die Phagen zugegeben.

Dazu wurde eine Vorratslösung aus 250 µM D-Aß in HFP (Hexafluorisopropanol) 1:100 in 0,8 ml TBST (mit 0,1 Gew.-% BSA) verdünnt und in ein mit Streptavidin beschichtetes Röhrchen überführt und für 15 Minuten bei Raumtemperatur leicht geschüttelt. Anschließend wurden 2x10¹¹ Phagen zugegeben und für 15 Min. leicht geschüttelt.

### Selektionsverfahren C:

Bei dem Selektionsverfahren C wurde das β-Amyloid-Peptid in Lösungsbedingungen überführt, die eine Verhinderung der Oligomerisierung gewährleisteten.

Dazu wurde eine Vorratslösung aus 20 µM D-Aβ in HFP (Hexafluorisopropanol) 1:10000 in 1,0 ml TBST (mit 0,1 Gew.-% BSA) verdünnt, in ein,mit Streptavidin beschichtetes Röhrchen überführt und für 15 Minuten bei Raumtemperatur leicht geschüttelt. Anschließend wurde zweimal mit 1,0 ml PBST (1 PBS (NaH₂PO₄ x H₂O und NaCl) enthaltend 0.05% Tween-20) gewaschen, 0,8 ml TBST mit 2x10¹¹ Phagen zugegeben und für 15 Minuten leicht geschüttelt.

Bei allen Selektionsverfahren A, B und C wurde identisch wie folgt weiterverfahren:

Die nichtbindenden Phagen wurden durch Abgießen entfernt. Um etwaige Streptavidin-bindende Phagen zu entfernen, wurde 0,1 mM Biotin für 5 Min. bei Raumtemperatur zugegeben. Danach wurde das Röhrchen 10 mal mit 1,5 ml TBST gewaschen.

Die an D-Aβ bindenden Phagen mit einem Peptid-Bibliothek-Insert wurden durch Zugabe von 0,8 ml 0,2 M Glycin-HCl (pH 2,2) für 10 Min. bei Raumtemperatur eluiert und in 0,2 ml 1M Tris-HCl (pH 9,1) zur Neutralisierung gegeben. Die Phagen-Titer wurden unter Verwendung einer Fraktion des Eluats zur Infektion von *E. coli* ER2738-Zellen unter Verwendung des Standardprotokolls (New England Biolabs) bestimmt. Zur Amplifizierung wurde das verbleibende Eluat in eine 20 ml Kultur (10 g/l Pepton (Roth, Karlsruhe), 5 g/l Hefeextrakt und 5g/l NaCl) von *E. coli* ER2738 am Anfang der logarithmischen Phase (optische Dichte bei 600 nm von 0,1) zugegeben und bei 37°C unter kräftigem Rühren für 4,5 Std. inkubiert. Die Phagen wurden durch Zentrifugation (30 Min. 5000 x g) der Kulturen und anschließendes Ausfällen der Phagen aus dem verbleibenden Überstand durch Zugabe von 7 ml M NaCl mit 20 Gew.-% Polyethylenglycol (PEG 8000) und Zentrifugation (30 Min. 5000 x g) nach 16 Std. Inkubation auf Eis geerntet. Das Pellet wurde wieder in 1 ml TBS (50 mM Tris-HCl pH 7,5, 150 mM NaCl) suspendiert, in ein Mikrozentrifugenröhrchen überführt und bei 10000 x g bei 4°C zentrifugiert, um die verbleibenden Zellen zu pelletieren. Der Überstand wurde mit 200 µl 2,5 M NaCl mit 20 Gew.-% Polyethylenglycol (PEG 8000) ausgefällt. Nach der Inkubation auf Eis für 1 Std. und Zentrifugation (20 Min., 13000 x g, 4°C) wurde das Pellet in 100 µl TBS suspendiert. Ein Aliquot der Phagen-Suspension wurde verwendet, um den Phagen-Titer nach der Amplifizierung zu bestimmen, um das Input-Volumen für den nächsten Schritt zu berechnen, das 2x10¹¹-Phagen entspricht.

Nach vier Runden der Selektion und Amplifizierung wurden bei dem Selektionsverfahren A 41 Klone zufällig für die DNA-Sequenzierung des Peptid-Bibliothek-Inserts ausgewählt. Nach der Übersetzung in Aminosäure-Sequenzen zeigten 23 Sequenzen eine sehr starke Sequenz-Ähnlichkeit (siehe Abbildung 1). Eine dieser Sequenzen war 21 mal in den 41 sequenzierten Proben enthalten, was darauf hindeutet, dass ein Peptid aus L-Aminosäuren mit dieser Sequenz sehr stark an D-Aβ bindet.

Die Aminosäuresequenzen der durch die Selektionsverfahren B und C ausgewählten Peptide sind in den Abbildungen 3 bzw. 4 dargestellt. Unter den nach dem Selektionsverfahren B erhaltenen Peptiden tritt die Sequenz GISWQQSHHLVA viermal auf, während von den 23 ausgewählten Klonen aus dem Selektionsverfahren C 17 eine sehr starke Sequenzähnlichkeit aufweisen. Auch bei diesen Beispielen weist das häufige Auftreten der jeweiligen Sequenzen auf eine sehr starke Bindung der Peptide aus L-Aminosäuren mit dieser Sequenz an D-Aβ hin.

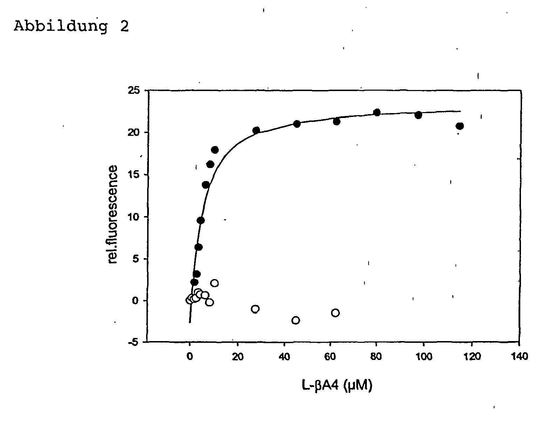

Um zu überprüfen, ob ein aus D-Aminosäuren bestehendes Peptid mit exakt derselben Sequenz an L-Aβ bindet, wurde ein entsprechendes chemisch synthetisiertes Peptid mit der Sequenz QSHYRHISPAQV (D-Pep) mit einer Fluorescein-Markierung an dessen C-Terminus für Fluoreszenz-Titrations-Untersuchungen verwendet. Die Abbildung 2 zeigt die Änderung der D-Pepabhängigen Fluoreszenz als Funktion der L-Aβ-Konzentration. Unter Annahme einer einfachen bimolekularen Reaktion zwischen L-Aβ und D-Pep führte die Analyse durch nichtlineare Kurvenanpassung (Müller et al., Biochemistry 1991, 30, 3709-3715) zu einem K_{D}-Wert von 4 µM.

Fluorescein-Isothiocyanat (FITC-I, Sigma) als Kontrolle bindet nicht an L-Aß.

### Abbildungen

Abbildung 1: Aminosäure-Sequenzen von nach dem Selektionsverfahren A erhaltenen Peptiden.
Abbildung 2: Bindung von L-Aβ an 0,8 µM D-Pep (•) und als Kontrolle an 1,0 µM Fluorescein (o) als Funktion der L-Aβ-Konzentration. Die Werte ergeben sich aus der Differenz der Fluoreszenz mit L-Aβ gelöst in HFP und HFP ohne L-Aβ. Unter Annahme einer einfachen bimolekularen Reaktion zwischen L-Aβ und dem D-Pep-Peptid ergab die Analyse durch nichtlineare Kurvenanpassung (durchgezogene Linie) einen K_{D}-Wert von 4 µM.
Abbildung 3: Aminosäure-Sequenzen von nach dem Selektionsverfahren B erhaltenen Peptiden.
Abbildung 4: Aminosäure-Sequenzen von nach dem Selektionsverfahren C erhaltenen Peptiden.

## Patentansprüche

1. Peptid,
**dadurch gekennzeichnet, dass** es im Wesentlichen aus D-Aminosäuren besteht und an Monomere und/oder Oligomere und/oder Fibrillen des β-Amyloid-Peptids mit einem K_{D}-Wert von höchstens 10 µM, vorzugsweise von höchstens 6 µM und besonders bevorzugt von höchstens 4 µM bindet und dass es ein aus D-Aminosäuren bestehendes Sequenzmotiv ausgewählt aus der Gruppe bestehend aus:
a) SHYRHISP,
b) GISWQQSHHLVA, oder
c) PRTRLHTH
umfasst.

2. Peptid nach Anspruch 1,
**dadurch gekennzeichnet, dass** die an der Bindung an das β-Amyloid-Peptid beteiligten Aminosäuren ausschließlich D-Aminosäuren sind.

3. Peptid nach Anspruch 1 oder 2,
**dadurch gekennzeichnet, dass** es ausgewählt ist aus der Gruppe, bestehend aus Peptiden mit den D-Aminosäure-Sequenzen:
a) QSHYRHISPAQV;
b) QSHYRHISPDQV;
c) QSHYRHISPAR;
d) KSHYRHISPAKV;
e) RPRTRLHTHRNR;
f) RPRTRLHTHRTE;
g) KPRTRLHTHRNR; und
h) Sequenzen, die die Sequenzen a) bis g) umfassen.

4. Peptid nach einem der vorangehenden Ansprüche,
**dadurch gekennzeichnet, dass** es ferner wasserlösliche Gruppen umfasst.

5. Peptid nach einem der vorangehenden Ansprüche,
**dadurch gekennzeichnet, dass** es ferner Antikörper umfasst.

6. Peptid nach einem der vorangehenden Ansprüche,
**dadurch gekennzeichnet, dass** es ferner für die Verwendung in Bild- oder anderen Signal-gebenden Verfahren geeignete Gruppen umfasst.

7. Peptid nach einem der vorangehenden Ansprüche,
**dadurch gekennzeichnet, dass** es ferner Gruppen umfasst, die das Wiederausscheiden des Peptids, frei oder gebunden an Aβ, aus dem Körper beschleunigen.

8. Verwendung der Peptide nach einem der Ansprüche 1 bis 7 zur Herstellung eines Mittels zur Diagnose der Alzheimer-Demenz bei lebenden Individuen.

9. Verwendung nach Anspruch 8, wobei die Peptide als Kontrastmittel zur Detektion von Amyloidplaques eingesetzt werden.

10. Verwendung nach Anspruch 8 oder 9, wobei die Peptide radioaktiv markiert werden.

11. Verwendung nach einem der Ansprüche 8 bis 10, wobei die Diagnose mittels PET erfolgt

12. Verwendung nach Anspruch 8, wobei die Detektion mittels Kernspintomographie oder Nah-Infrarot-Detektion erfolgt.

13. Verwendung der Peptide nach einem der Ansprüche 1 bis 7 zur Herstellung eines Medikaments zur Vorbeugung und/oder Therapie der Alzheimer-Demenz.

14. Verwendung der Peptide nach einem der Ansprüche 1 bis 7 zur Herstellung einer Zusammensetzung zur Untersuchung des Zusammenhangs zwischen pathologischen Befunden und Krankheitssymptomen bei der Alzheimer-Demenz.

## Claims

1. Peptide,
**characterized in that** it essentially consists of D-amino acids and binds to monomers and/or oligomers and/or fibrils of the β-amyloid peptide having a K_{D} value of a maximum of 10 µM, more preferred of a maximum of 6 µM, and especially preferred of a maximum of 4 µM and that it comprises a sequence motive consisting of D-amino acids selected from the group consisting of:
a) SHYRHISP,
b) GISWQQSHHLVA, or
c) PRTRLHTH.

2. Peptide according to claim 1,
**characterized in that** the amino acids participating in the binding to the β-amyloid peptide are exclusively D-amino acids.

3. Peptide according to claim 1 or 2,
**characterized in that** it is selected from the group consisting of peptides with the D-amino acid sequences:
a) QSHYRHISPAQV;
b) QSHYRHISPDQV;
c) QSHYRHISPAR;
d) KSHYRHISPAKV;
e) RPRTRLHTHRNR;
f) RPRTRLHTHRTE;
g) KPRTRLHTHRNR; and
h) sequences which comprise the sequences a) to g).

4. Peptide according to any of the preceding claims,
**characterized in that** it further comprises water soluble groups.

5. Peptide according to any of the preceding claims,
**characterized in that** it further comprises antibodies.

6. Peptide according to any of the preceding claims,
**characterized in that** it further comprises groups suitable for use in image or other signal-transmitting methods.

7. Peptide according to any of the preceding claims,
**characterized in that** it further comprises groups accelerating the re-excretion of the peptide, free or bound to Aβ, from the body.

8. Use of the peptides according to any of claims 1 to 7, for the production of an agent for diagnosis of Alzheimer's disease in living individuals.

9. Use according to claim 8, wherein the peptides are used as contrast agent for the detection of amyloid plaques.

10. Use according to claim 8 or 9, wherein the peptides are to be radioactively labeled.

11. Use of any of claims 8 to 10, wherein the diagnosis is carried out by means of PET.

12. Use according to claim 8, wherein the detection is carried out by means of magnetic resonance imaging or near-infrared detection.

13. Use of the peptide according to any of claims 1 to 7 for the production of a medicament for prophylaxis and/or treatment of Alzheimer's disease.

14. Use of the peptide according to any of claims 1 to 7 for the production of a formulation for the investigation of a correlation between pathological findings and disease symptoms of Alzheimer's disease.

## Revendications

1. Peptide, **caractérisé en ce qu'**il est essentiellement constitué par des D-aminoacides et se lie à des monomères et/ou des oligomères et/ou des fibrilles du peptide β-amyloïde avec une valeur K_{D} d'au plus 10 µM, de préférence d'au plus 6 µM et de manière particulièrement préférée d'au plus 4 µM et qu'il comprend un motif de séquence constitué par des D-aminoacides choisi dans le groupe formé par :
a) SHYRHISP,
b) GISWQQSHHLVA, ou
c) PRTRLHTH.

2. Peptide selon la revendication 1, **caractérisé en ce que** les aminoacides qui interviennent dans la liaison au peptide β-amyloïde sont exclusivement des D-aminoacides.

3. Peptide selon la revendication 1 ou 2, **caractérisé en ce qu'**il est choisi dans le groupe formé par les peptides présentant les séquences de D-aminoacides :
a) QSHYRHISPAQV;
b) QSHYRHISPDQV;
c) QSHYRHISPAR;
d) KSHYRHISPAKV;
e) RPRTRLHTHRNR;
f) RPRTRLHTHRTE;
g) KPRTRLHTHRNR; et
h) les séquences qui comprennent les séquences a) à g).

4. Peptide selon l'une quelconque des revendications précédentes, **caractérisé en ce qu'**il comprend en outre des groupes solubles dans l'eau.

5. Peptide selon l'une quelconque des revendications précédentes, **caractérisé en ce qu'**il comprend en outre des anticorps.

6. Peptide selon l'une quelconque des revendications précédentes, **caractérisé en ce qu'**il comprend en outre des groupes appropriés pour l'utilisation dans des procédés d'imagerie ou d'autres procédés émettant un signal.

7. Peptide selon l'une quelconque des revendications précédentes, **caractérisé en ce qu'**il comprend en outre des groupes qui accélèrent la ré-excrétion du peptide libre ou lié au peptide Aβ du corps.

8. Utilisation des peptides selon l'une quelconque des revendications 1 à 7 pour la préparation d'un agent destiné au diagnostic de la démence d'Alzheimer chez des individus vivants.

9. Utilisation selon la revendication 8, les peptides étant utilisés comme agent de contraste pour la détection de plaques d'amyloïde.

10. Utilisation selon la revendication 8 ou 9, les peptides étant marqués radioactivement.

11. Utilisation selon l'une quelconque des revendications 8 à 10, le diagnostic étant réalisé par PET (tomographie par émission de positrons).

12. Utilisation selon la revendication 8, la détection étant réalisée par tomographie de spin nucléaire ou par détection de l'infrarouge proche.

13. Utilisation des peptides selon l'une quelconque des revendications 1 à 7 pour la préparation d'un agent destiné à prévenir et/ou à la thérapie de la démence d'Alzheimer.

14. Utilisation des peptides selon l'une quelconque des revendications 1 à 7 pour la préparation d'une composition destinée à étudier la relation entre des conclusions pathologiques et des symptômes de maladies dans le cas de la démence d'Alzheimer.
